# EUROPÄISCHE PATENTANMELDUNG

(11) **EP 1 950 183 A1**
(43) Veröffentlichungstag der Anmeldung: **30.07.2008**
(21) Anmeldenummer: 08000331.2
(22) Anmeldetag: 10.01.2008
(51) Int. Cl.: C03B 19/06, C04B 35/14, C03B 5/43

(54) **Sintern von Quarzglas zu kristallines SiO2 enthaltenden Formkörpern**

(30) Priorität: 23.01.2007 DE 102007004424
(71) Anmelder: Schott AG, 55122 Mainz (DE)
(72) Erfinder: Borens, Manfred, 55126 Mainz (DE); Von Westernhagen, Karsten, Dr., 55270 Essenheim (DE); Merolla, Stefano, 55129 Mainz (DE); Wasem, Gerald, 67822 Schmalfelderhof (DE); Postrach, Stefan, 55126 Mainz (DE)
(74) Vertreter: Herden, Andreas F.

(57) **Zusammenfassung**

Für ein einfaches und schnelles Herstellen eines Formkörpers aus Quarzglas und einen Formkörper, welcher auch bei hohen Temperaturen kaum zu Verunreinigungen einer mit dem Formkörper in Kontakt stehenden Schmelze führt, stellt die Erfindung ein Verfahren mit den Schritten:
a) Bereitstellen von Quarzglas in Form von im wesentlichen amorphen SiO₂-Körnern, von denen maximal 5 % einen Durchmesser größer als 15 mm aufweisen,
b) Zugabe von Wasser zu den Quarzglaskörnern zum Herstellen einer Schlickermasse,
c) Gießen der Schlickermasse in eine Form, die einen Hohlkörper mit der inversen Gestalt des herzustellenden Formkörpers umfaßt,
d) Trocknen der Schlickermasse zu einem Vor-Körper,
e) Sintern des Vor-Körpers bei einer Sintertemperatur, bei welcher zumindest ein Teil des Quarzglases von der amorphen in eine kristalline Modifikation umgewandelt wird,
f) Abkühlen des gesinterten Formkörpers auf eine Temperatur unter 300°C derart, daß ein Gefüge entsteht, welches kristallines SiO₂ enthält,
zur Verfügung sowie einen Formkörper, welcher mindestens 99,0 mol.-% an SiO₂ umfaßt.

## Beschreibung

Die Erfindung betrifft ein Verfahren zum Herstellen eines Formkörpers aus Quarzglas durch Sintern, einen Formkörper und die Verwendung eines solchen Formkörpers.

Im Speziellen betrifft die Erfindung die Verarbeitung von Schmelzen von Glas, Metallen, Halbmetallen und ähnlichen Materialien bei hohen Temperaturen von mehreren 100°C. Die Verarbeitung kann beispielsweise das Herstellen einer Schmelze aus Rohmaterial, bei Glas insbesondere Scherben und/oder Gemenge, sowie Läutern, Homogenisieren und weitere Schritte wie Zuführen zur Formgebung umfassen. Bei allen derartigen Verfahrensschritten werden Behälter wie Tiegel, Schmelzwannen, Läuterwannen, Rohrleitungen, Rührer und andere Aggregate eingesetzt, von denen im Betrieb Flächen mit der Schmelze in Berührung kommen.

Von einem Material für derartige Bauteile wird ein hoher Widerstand gegen einen chemischen Angriff infolge des Kontakts mit der Schmelze, das heißt eine hohe Korrosionsbeständigkeit, gefordert, damit die Verunreinigung der Schmelze durch den Eintrag von Teilchen des Materials möglichst nicht auftreten.

In DE 102 44 040 der Anmelderin wird für die Glasherstellung ein Sinterquarzgut vorgeschlagen, welches einen Zweischichtaufbau aufweist. Cristobalit ist eine Kristallmodifikation von SiO₂ und bietet den Vorteil höherer Korrosionsbeständigkeit als amorphes Kieselglas. Gemäß DE 102 44 040 ist bei der Herstellung des Materials durch Schlickerguß von Kieselglasteilchen darauf zu achten, daß kein beziehungsweise nur wenig Cristobalit gebildet wird, da sich bei etwa 270°C beta-Cristobalit in alpha-Cristobalit unter Volumenabnahme von mindestens 2 % umwandelt. Diese Volumenänderung wird als das Bauteil zerstörend angesehen.

Um beim Einsatz des Bauteils die hohe Korrosionsbeständigkeit des kristallinen SiO₂ nutzen zu können, wird die Teilchengröße der SiO₂-Körner zumindest im äußeren Bereich des Bauteils so gewählt, daß im Betrieb die Cristobalisierung schneller erfolgt als der Abtrag des Kieselglases durch Korrosion. Eine äußere Schicht des Materials für den Einsatz im Kontakt mit einer Glasschmelze besteht aus Kieselglaskörnern mit einem Durchmesser unter 40 Mikrometern. Diese kleinen Kieselglaskörner werden eingesetzt, damit im Betrieb bei entsprechend hohen Temperaturen eine möglichst schnelle Entglasung des Kieselglases unter Umwandlung in Cristobalit erfolgt.

Die Herstellung eines zweischichtigen Formkörpers ist jedoch arbeits- und damit kostenintensiv. Zudem muß der Sintervorgang bei ausreichend niedrigen Temperaturen durchgeführt werden, um eine Kristallisation weitgehend zu vermeiden. Der Sintervorgang ist daher zeitaufwendig. Des Weiteren muß beim Einsatz des Formkörpers im Kontakt mit einer Schmelze beim Betrieb bei hohen Temperaturen die dann auftretende Schwindung durch die Umwandlung des amorphen Quarzglases in kristalline Modifikationen beachtet werden.

Es ergibt sich daher eine Aufgabe der Erfindung, ein einfaches und schnelles Verfahren zum Herstellen eines Formkörpers aus Quarzglas durch Sintern zu schaffen. Eine weitere Aufgabe der Erfindung ist es, einen Formkörper bereit zu stellen, welcher auch bei hohen Temperaturen kaum zu Verunreinigungen einer Schmelze führt, welche mit dem Formkörper in Kontakt steht.

Diese Aufgaben werden mit der Erfindung auf überraschend einfache Weise gelöst mit einem Verfahren und einem Formkörper gemäß den unabhängigen Ansprüchen. Vorteilhafte Weiterbildungen sind Gegenstand der jeweils zugeordneten Unteransprüche.

Die Erfindung stellt ein Verfahren zum Herstellen eines Formkörpers zur Verfügung mit den Schritten:
a) Bereitstellen von Quarzglas in Form von im wesentlichen amorphen SiO₂-Körnern, von denen 5 %, bevorzugt maximal 3 %, besonders bevorzugt maximal 2 % einen Durchmesser größer als 15 mm, bevorzugt größer als 10 mm, besonders bevorzugt größer als 6 mm aufweisen,
b) Zugabe von Wasser zu den Quarzglaskörnern zum Herstellen einer Schlickermasse,
c) Gießen der Schlickermasse in eine Form, die einen Hohlkörper mit der inversen Gestalt des herzustellenden Formkörpers umfaßt,
d) Trocknen der Schlickermasse zu einem Vor-Körper,
e) Sintern des Vor-Körpers bei einer Sintertemperatur, bei welcher zumindest ein Teil des Quarzglases von der amorphen in eine kristalline Modifikation, insbesondere in beta-Cristobalit, umgewandelt wird,
f) Abkühlen des gesinterten Formkörpers auf eine Temperatur unter 300°C, bevorzugt unter 270°C, derart, daß ein Gefüge entsteht, welches kristallines SiO₂ enthält.

### Beispielsweise kann dabei in weiteren Einzelschritten vorgegangen werden, die unten näher erläutert werden.

Überraschenderweise und entgegen den Erwartungen aus der DE 102 44 04 wird der Formkörper durch den Abkühlvorgang nach dem Brennen nicht zerstört, obwohl Cristobalit bereits während des Herstellens des Bauteils entsteht. Die Umwandlung des beta-Cristobalits zu alpha-Cristobalit ist mit einer Volumenabnahme zwischen 2 und 2,8 % verbunden. Selbst wenn gemäß der Erfindung jedoch keinerlei Stabilisatoren zugegeben werden und damit vorteilhafterweise die Reinheit des Materials erhalten bleibt, bleibt der Formkörper gemäß der Erfindung stabil und läßt sich problemlos weiterverarbeiten, da die erfindungsgemäße Größe der eingesetzten Quarzglaskörner zu einem Gefüge im gesinterten Formteil führt, welches die bei der Umwandlung zwischen den Kristallmodifikationen entstehenden Spannungen toleriert.

Damit bietet die Erfindung den Vorteil einer besseren Korrosionsbeständigkeit insbesondere gegenüber Glasschmelzen, weil durch die Kristallbildung bereits bei der Herstellung die Umwandlung von amorphem zu kristallinem SiO₂ im späteren Einsatz des Bauteils schneller startet, denn es sind bei der Herstellung bereits Kristallkeime eingebaut worden.

In Schritt c) kann beispielsweise eine Gipsform verwendet werden. Die Flächen der Form, welche mit dem Formteil in Kontakt kommen, können je nach Anforderungen an die Oberflächengüte des herzustellenden Bauteils bearbeitet werden. Das Brennen des Vor-Körpers zum Zweck des Sinterns in Schritt e) auch nach Ausformen des Vor-Körpers erfolgen. Je nachdem, wie die äußere Form des Vor-Körpers gestaltet ist und welchen Belastungen, beispielsweise durch sein Eigengewicht, der Vor-Körper infolgedessen standhalten kann, kann ein Vorsintern in der Form erfolgen.

In einer bevorzugten Weiterbildung der Erfindung wird in Schritt a) im wesentlichen reines Quarzglas in amorpher Form mit einem Gehalt von mindestens 99,0 Gew.-%, bevorzugt mindestens 99,5 Gew.-%, besonders bevorzugt mindestens 99,9 Gew.-% an SiO₂ eingesetzt. Das in Schritt a) eingesetzte SiO2 kann bis zu 3 % kristallines SiO₂ enthalten.

Vorzugsweise wird in Schritt b) im wesentlichen alkalifreies Wasser verwendet.

Das Sintern des Vor-Körpers in Schritt e) kann gemäß der Erfindung bei relativ hohen Temperaturen durchgeführt werden, so daß außer der Ausbildung von Materialbrücken zwischen den Quarzglaskörnern eine Kristallbildung einsetzt. Dazu ist vorgesehen, das Sintern bei einer Temperatur von mindestens 1080°C, vorzugsweise von mindestens 1120°C, bevorzugt von mindestens 1140°C, besonders bevorzugt von mindestens 1180°C durchzuführen. Durch die hohe Sintertemperatur gemäß der Erfindung kann zudem die Ofenregelung vereinfacht werden und/oder die Brenndauer gegenüber dem Brennen bei Sintertemperaturen von höchstens 1000°C deutlich verkürzt werden. Zudem erfolgt eine bessere Versinterung der Körner, welche eine geringere Restporosität nach dem Brennen, eine höhere Dichte, eine bessere mechanische Stabilität und eine geringere Schwindung aufgrund von Nachsintereffekten beim späteren Einsatz des Bauteils mit sich bringt.

In Abstimmung auf die Abmessungen des Vor-Körpers wird in einer bevorzugten Ausgestaltung der Erfindung die maximale Temperatur beim Sintern so gewählt, daß der herzustellende Formkörper eine homogene innere Struktur durch das Versintern des Quarzglases erhält und gleichzeitig vorteilhafterweise die Kristallbildung in den Außenbereichen des Formkörpers im gewünschten Ausmaß erfolgt. Nach Erkenntnissen der Erfinder hat sich beispielsweise eine maximale Temperatur beim Sintern von etwa 1285°C bei einer größten Wandstärke des Vor-Körpers von kleiner oder gleich etwa 85 mm als geeignet herausgestellt. Bei einer größten Wandstärke des Vor-Körpers von mehr als etwa 85 mm kann diese Temperatur beispielsweise auf etwa 1250°C gesenkt werden.

Durch das erfindungsgemäße Verfahren wird es möglich, einen Formkörper zu schaffen, welcher nahezu vollständig aus SiO₂ besteht und Kristalleinlagerungen aufweist. Die Kristalleinlagerungen können beim späteren Einsatz des Formkörpers in Kontakt mit einem sehr heißen Material, beispielsweise einer Glasschmelze, als Keime für weiteres Kristallwachstum in Form von Cristobalit dienen, welches eine höhere Korrosionsbeständigkeit aufweist als amorphes Quarzglas. Besonders vorteilhaft ist bei dem erfindungsgemäßen Formkörper die mit dem Gehalt an kristallinem SiO₂ verbundene höhere Dichte und geringere Porosität, welche zusätzlich zur mechanischen Stabilität des Bauteils beitragen, ohne daß Zuschlagsstoffe wie zum Beispiel Pulver aus reinem Silizium eingesetzt werden müßten.

Die Erfindung stellt somit einen Formkörper zur Verfügung, welcher insbesondere herstellbar oder hergestellt mit einem oben beschriebenen Verfahren ist und mindestens 99,0 mol.-%, bevorzugt mindestens 99,5 mol.-%, besonders bevorzugt mindestens 99,9 mol.-% an SiO₂ umfaßt.

In einer bevorzugten Ausführungsform der Erfindung umfaßt der Formkörper in seiner äußeren Schicht, welche von seiner Oberfläche aus gemessen eine Dicke von bis zu 40 mm aufweist, einen über die Dicke gemittelten Gehalt im Bereich von 2 Vol-% bis 5 Vol-%, bevorzugt von 2,5 Vol-% bis 4,5 Vol-%, besonders bevorzugt von 2,9 Vol.-% ± 0,1 Vol.-% kristallines SiO₂, insbesondere Cristobalit.

Der Gehalt an kristallinem SiO₂ kann bei Raumtemperatur als alpha-Cristobalit mittels Röntgenbeugung gemessen werden.

Um eine besonders hohe Korrosionsbeständigkeit des Formkörpers beim Einsatz im Kontakt mit sehr heißen Medien zu erreichen, ohne daß bei Temperaturwechseln, zum Beispiel durch Phasenübergänge, derartige Volumenänderungen eintreten, die zu Rissen im Formkörper führen, ist der Formkörper gemäß der Erfindung vor allem in den oberflächennahen Bereichen mit einem erhöhten Gehalt an kristallinem SiO₂ versehen.

In einer vorteilhaften Ausgestaltung umfaßt der Formkörper in seiner äußeren Schicht, welche von seiner Oberfläche aus gemessen eine Dicke von bis zu 20 mm aufweist, einen über die Dicke gemittelten Gehalt im Bereich von 2,3 Vol-% bis 5,5 Vol-%, bevorzugt im Bereich von 3,0 Vol-% bis 5,5 Vol-%, besonders bevorzugt von 3,5 Vol-% bis 4,5 Vol-%, besonders bevorzugt von 3,75 Vol.-% ± 0,1 Vol.-% kristallines SiO₂, insbesondere Cristobalit.

Besonders bevorzugt ist ein Formkörper, welcher in seiner äußeren Schicht, die von seiner Oberfläche aus gemessen eine Dicke von bis zu 10 mm aufweist, einen über die Dicke gemittelten Gehalt im Bereich von 2,3 Vol-% bis 6,5 Vol-%, bevorzugt im Bereich von 3,5 Vol-% bis 6,5 Vol-%, besonders bevorzugt von 4 Vol-% bis 5 Vol-%, besonders bevorzugt von 4,6 Vol.-% ± 0,1 Vol.-% kristallines SiO₂, insbesondere Cristobalit umfaßt. In einer vorteilhaften Weiterbildung ist vorgesehen, daß der Formkörper in seiner äußeren Schicht, welche von seiner Oberfläche aus gemessen eine Dicke von bis zu 5 mm aufweist, einen über die Dicke gemittelten Gehalt im Bereich von 2,3 Vol-% bis 7,5 Vol-%, bevorzugt im Bereich von 4,0 Vol-% bis 7,5 Vol-%, besonders bevorzugt von 4,5 Vol-% bis 6,5 Vol-%, besonders bevorzugt von 5,1 Vol.-% ± 0,1 Vol.-% kristallines SiO₂, insbesondere Cristobalit umfaßt.

Nach einer bevorzugten Ausführungsform umfaßt der Formkörper in seiner äußeren Schicht, welche von seiner Oberfläche aus gemessen eine Dicke von bis zu 2 mm aufweist, einen über die Dicke gemittelten Gehalt im Bereich von 2,3 Vol-% bis 8,0 Vol-%, bevorzugt im Bereich von 4,3 Vol-% bis 8,0 Vol-%, besonders bevorzugt von 4,8 Vol-% bis 6,8 Vol-%, besonders bevorzugt von 5,3 Vol.-% ± 0,1 Vol.-% kristallines SiO₂, insbesondere Cristobalit. Vorteilhafterweise umfaßt der Formkörper in seiner äußeren Schicht, welche von seiner Oberfläche aus gemessen eine Dicke von bis zu 1 mm aufweist, einen über die Dicke gemittelten Gehalt im Bereich von 2,5 Vol-% bis 8,5 Vol-%, bevorzugt im Bereich von 4,5 Vol-% bis 8,5 Vol-%, besonders bevorzugt von 5 Vol-% bis 6,3 Vol-%, besonders bevorzugt von 5,4 Vol.-% ± 0,1 Vol.-% kristallines SiO₂, insbesondere Cristobalit.

In einer bevorzugten Ausgestaltung ist vorgesehen, daß der Formkörper in seiner äußeren Schicht, welche von seiner Oberfläche aus gemessen eine Dicke von bis zu 0,3 mm aufweist, einen über die Dicke gemittelten Gehalt im Bereich von 2,7 Vol-% bis 9,5 Vol-%, bevorzugt im Bereich von 4,7 Vol-% bis 9,5 Vol-%, besonders bevorzugt von 5,1 Vol-% bis 6,8 Vol-%, besonders bevorzugt von 5,5 Vol.-% ± 0,1 Vol.-% kristallines SiO₂, insbesondere Cristobalit umfaßt. Besonders bevorzugt ist ein Formkörper, welcher in einer Schicht, welche von seiner Oberfläche aus gemessen zwischen einer Tiefe von 20 mm und einer Tiefe von bis zu 40 mm liegt und somit eine Dicke von bis zu 20 mm aufweist, einen über die Dicke gemittelten Gehalt im Bereich von 1 Vol-% bis 3 Vol-%, bevorzugt von 1,5 Vol-% bis 2,5 Vol-%, besonders bevorzugt von 2 Vol.-% ± 0,1 Vol.-% kristallines SiO₂, insbesondere Cristobalit umfaßt.

Neben dem Gehalt an kristalliner Phase zur Erhöhung der Korrosionsbeständigkeit des Formkörpers weist dieser gemäß der Erfindung vorteilhafterweise auch eine verbesserte mechanische Stabilität auf. So liegt die Dichte von Sinterquarzgut, das im Schlickerverfahren hergestellt wird, in der Regel zwischen 1,80 und 2,05 g/cm³. Durch die höhere Brenntemperatur und das bewußte Herbeiführen der Cristobalitbildung beim Sintern kann mit der Erfindung jedoch eine höhere Dichte erreicht werden. Der erfindungsgemäße Formkörper weist zumindest in einem Bereich, insbesondere in einem Bereich seiner äußeren Schicht, welche von der Oberfläche des Formkörpers aus gemessen eine Dicke von bis zu 20 mm aufweist, eine über die Dicke gemittelte Dichte im Bereich von 1,92 g/cm³ bis 2,12 g/cm³ auf.

Die offene Porosität von Sinterquarzgut, welches im Schlickerverfahren hergestellt wird, liegt zwischen 10,5 und 15 Vol.-%. Hier wird insbesondere durch das erfindungsgemäße Herstellungsverfahren ein Formkörper geschaffen, welcher zumindest in einem Bereich, insbesondere in einem Bereich seiner äußeren Schicht, welche von der Oberfläche des Formkörpers aus gemessen eine Dicke von bis zu 20 mm aufweist, eine über die Dicke gemittelte offene Porosität im Bereich von weniger als 12 % , bevorzugt von weniger als 9 % , besonders bevorzugt von 7,5 % aufweist.

Die Eigenschaften des erfindungsgemäßen Formkörpers machen diesen besonders geeignet zur Verwendung als Tiegel oder Teil eines Tiegels zum Einsatz in Kontakt mit einer Schmelze zumindest eines Metalls und/oder Halbmetalls und/oder Halbleiters und/oder Glases. Ebenso wie für einen Tiegel sieht die Erfindung die Verwendung des Formkörpers für eine Schmelzwanne und/oder eine Läuterwanne und/oder eine Rohrleitung und/oder einen Rührer und/oder ein anderes Aggregat im Einsatz bei der Verarbeitung von Schmelzen vor.

Die Erfindung wird nachfolgend anhand von Ausführungsbeispielen und unter Bezugnahme auf die beigefügten Zeichnungen näher erläutert. Es zeigen:
- Fig. 1: Schematische Darstellung des Ablaufs des Verfahrens,
- Fig. 2: Auftragung der Längenänderung beim Aufheizen und Abkühlen des Sinterquarzgutes während des Herstellens des Formkörpers,
- Fig. 3: schematische Darstellung eines vergrößerten Ausschnitts X aus einem Formkörpers,
- Fig. 4: Mikroskopaufnahme eines Schliffes aus einem Formkörper mit Cristobalit-Anteil gemäß der Erfindung,
- Fig. 5: Mikroskopaufnahme eines Schliffes aus einem Formkörper, der bei üblicher Temperaturführung gesintert wurde, und
- Fig. 6: schematische Darstellung eines Formkörpers.

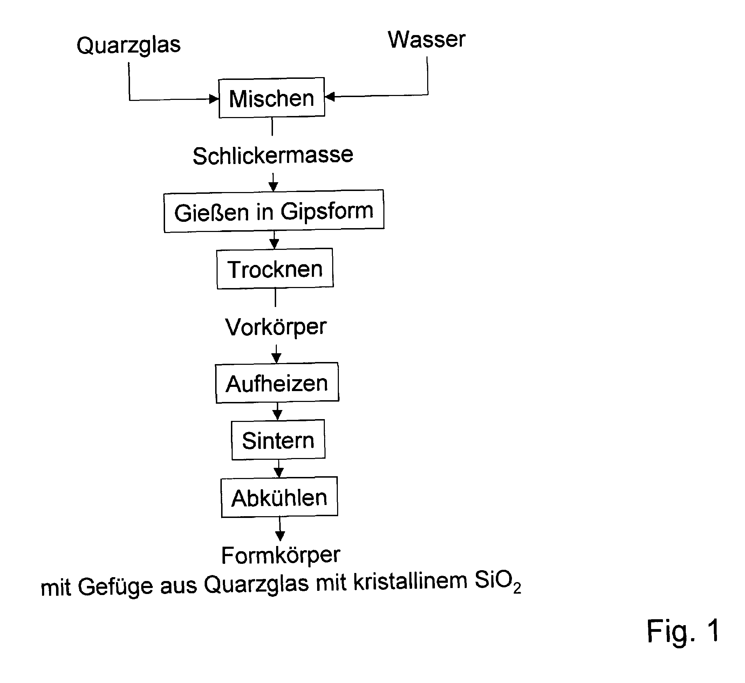

Um einen Formkörper mit einem Gefüge aus versinterten, amorphen Quarzglaskörnern, in welches SiO₂ in kristalliner Form, insbesondere in Form von Cristobalit, eingebettet ist, wird durch Mischen von Quarzglaskörnern mit Wasser eine Schlickermasse hergestellt (siehe Figur 1).

Für die Schlickermasse wird reines Quarzglas verwendet. Es wird kein Stabilisator wie zum Beispiel Phosphat zur Unterdrückung der Cristobalit-Bildung zugegeben. Es kommen verschiedene Klassen für die Reinheit des Quarzglases in Frage, nämlich mindestens 99,0 % SiO₂, besser mindestens 99,5 % SiO₂ und besonders geeignet ist Quarzglas mit einer Reinheit von mindestens 99,9 % SiO₂. Die restlichen Bestandteile sind Metalloxide, die mit dem Rohmaterial (Quarzglas/Kieselglas) eingeschleppt werden oder während der Verarbeitung durch Kontakt mit den Konstruktionswerkstoffen der Anlagen in das Produkt gelangen. Beispiele für derartige Metalloxide sind Al₂O₃, Na₂O, Fe-Oxide, TiO und weitere. Weitere Verunreinigungen können durch das verwendete Wasser eingebracht werden (z.B. Na-, Mg,- Ca-Salze).

Das reine Quarzglas wird gemahlen, dann werden verschiedene Korngrößenklassen separiert. Für die hier genannten Bauteile werden Körner beziehungsweise Bruchstücke mit einer Größe über 8 mm ausgesiebt. Zwar hat ein geringer Anteil, bis etwa 2 %, an Körnern mit einem mittleren Durchmesser über 8 mm noch keinen schädlichen Einfluß auf das Materialverhalten; bei einem Anteil über 2 % wird aber die Fließfähigkeit der Schlickermasse negativ beeinflußt. Die Herstellung dünnwandiger Bauteile mit Wandstärken unter 25 mm ist dann nur äußerst schwierig möglich. Weil die größeren Körner keinen Vorteil bringen, werden sie daher möglichst komplett ausgesiebt.

Für die Schlickermasse wird alkalifreies Wasser verwendet. In der Schlickermasse haben die Quarzglaskörner an ihrer Oberfläche eine Wasserschicht, wobei die Mengenverhältnisse von Quarzglas zu Wasser derart eingestellt werden, daß die Schlickermasse ein strukturviskoses, insbesondere rheopexes Fließverhalten hat.

Die Schlickermasse wird in eine Gipsform gegossen. Die Gipsform nimmt Wasser aus der Schlickermasse auf, so daß bereits dadurch ein Trocknen des Körpers aus Schlickermasse in der Gipsform erfolgt. Das Trocknen kann bei Raumtemperatur unter Atmosphärendruck allein durch die fortschreitende Wasseraufnahme der Gipsform durchgeführt werden, bis ein hinreichend stabiler Vor-Körper hergestellt ist. Das Trocknen kann durch Erhöhen der Temperatur und/oder Absenken des Drucks beschleunigt werden.

Der Vor-Körper wird dann auf eine Temperatur aufgeheizt, bei welcher die Quarzglaskörner versintern und teilweise ein Übergang von der amorphen in eine kristalline Phase erfolgt. Anschließend erfolgt ein Abkühlen. Als Endprodukt des Herstellungsverfahrens liegt ein Formkörper mit einem Gefüge aus Quarzglas mit kristallinem SiO₂ vor.

Die Sintertemperatur beziehungsweise Brenntemperatur liegt über 1080°C, besser über 1120°C. Bevorzugt ist eine Sintertemperatur beziehungsweise Brenntemperatur von über 1140°C, noch besser über 1180°C. Die Maximaltemperatur bei Brennen wird unter Abstimmung auf die größte Wanddicke des Bauteils gewählt. Als geeignet hat sich eine maximale Brenntemperatur von 1250°C bei größten Wanddicken von mehr als 85 mm und von 1285°C bei größten Wanddicken kleiner oder gleich 85 mm herausgestellt.

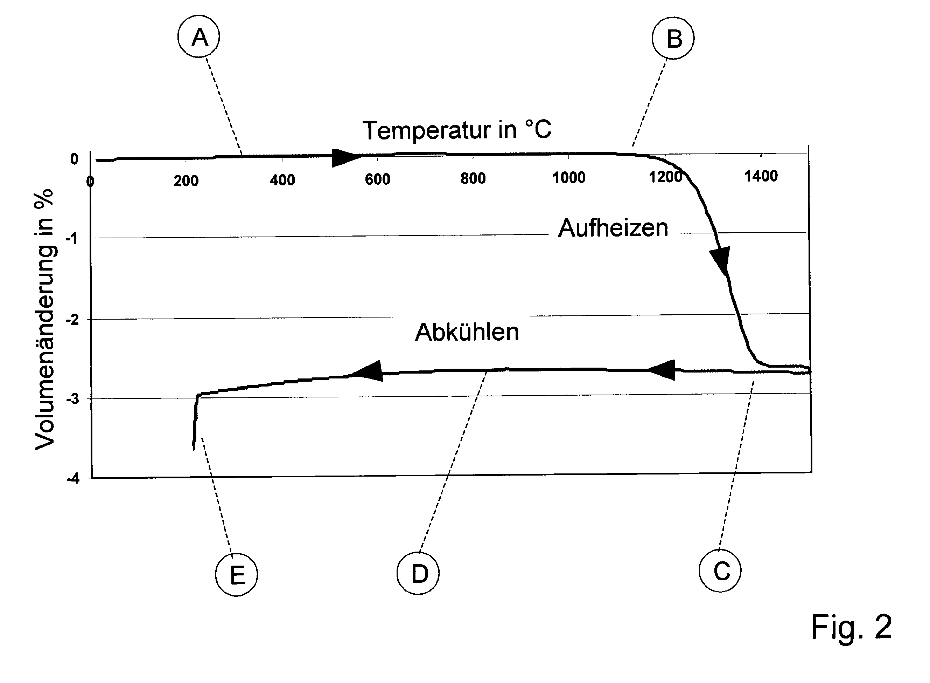

In Figur 2 ist die mit der Temperaturführung beim Aufheizen, Sintern und Abkühlen verbundene Volumenänderung des Formkörpers aufgetragen. Die im einzeln durchgeführten Schritte zeigt folgende Tabelle:

| | **Beschreibung** | **Bemerkung** |
|---|---|---|
| **Schritt 1** | von Raumtemperatur auf 90°C bis 120°C mit 10 bis 30 K/min | |
| **Schritt 2** | Haltezeit (abhängig von Wanddicke) 12 Std. bis 96 Std. | Trocknen |
| **Schritt 3** | weiter aufheizen mit 1 K/min bis 30 K/min auf 1030°C bis 1230 | ggf. weitere Haltezeiten in Abhängigkeit von der Wanddicke °C einbauen; z.B.: 24 Std bei 300 °C |
| **Schritt 4** | Haltezeit (abhängig von Wanddicke) 0,25 Std. bis 24 Std. | |
| **Schritt 5** | weiter aufheizen auf die maximale Brenntemperatur zwischen 1100°C und 1285°C mit ca. 1 K/min | in Ausnahmefällen: Maximaltemperatur bis 1320°C |
| **Schritt 6** | Haltezeit bei der Maximaltemperatur von 1 Std. bis 32 Std. | |
| **Schritt 7** | Abkühlen auf 380°C; maximale Abkühlgeschwindigkeit: 350 K/min | kann ungeregelt erfolgen / Abschalten der heizleistung; Abkühlrate ergibt sich aus Wärmeverlust (beispielsweise abhängig von der Ofenisolation) |
| **Schritt 8** | Haltezeit; abhängig von der Wanddicke; max. 4 Std. | für Bauteile mit Wanddicke unter 40 mm: keine Haltezeit; bei Wanddicke >120 mm: 4 Std. |
| **Schritt 9** | Abkühlen auf 250°C; Abkühlrate: zwischen 1K/min und 25 K/min | |
| **Schritt 10** | Haltezeit in Abhg. von der Wanddicke; max. 1 Std. | für Bauteile mit Wanddicke unter 40 mm: keine Haltezeit; bei Wanddicke >120 mm: 4 Std. |
| **Schritt 11** | Abkühlen auf Raumtemperatur; keine geregelte Abkühlung nötig; Bauteil kann sofort aus Ofen entnommen werden | |

Während des Aufheizens ist bis zu einer Temperatur von etwas über 1000°C die thermische Ausdehnung vernachlässigbar gering. Während des Aufheizens bleibt das Volumen nahezu konstant (Bereich A im Diagramm).

Ab etwa 1000°C beginnt die Versinterung der amorphen Quarzglaskörner; ab etwa 1100°C erfolgt eine Umwandlung der amorphen Glasphase in kristallines Beta-Christoballit. Damit ist eine Volumenverringerung verbunden (Bereich B). Das Aufheizen für die Versinterung und Kristallbildung wird bis zu einer Temperatur von über 1300°C fortgesetzt.

Sobald die Versinterung und Umwandlung in die kristalline Modifikation im gewünschten Maße abgeschlossen ist, wird das Bauteil abgekühlt (ab Bereich C). Das Bauteil, das nach diesem Prozeß hergestellt wurde, kann ohne Veränderung auch bei sehr hohen Temperaturen bis hin zu seiner Schmelztemperatur eingesetzt werden. Bis zu einer Temperatur von etwa 270°C erfolgt die Abkühlung ohne wesentliche Volumenänderung (Bereich D). Ab etwa 270°C wird Beta-Cristobalit in Alpha-Cristobalit unter sofortiger Volumenabnahme von etwa 3 % umgewandelt (Bereich E).

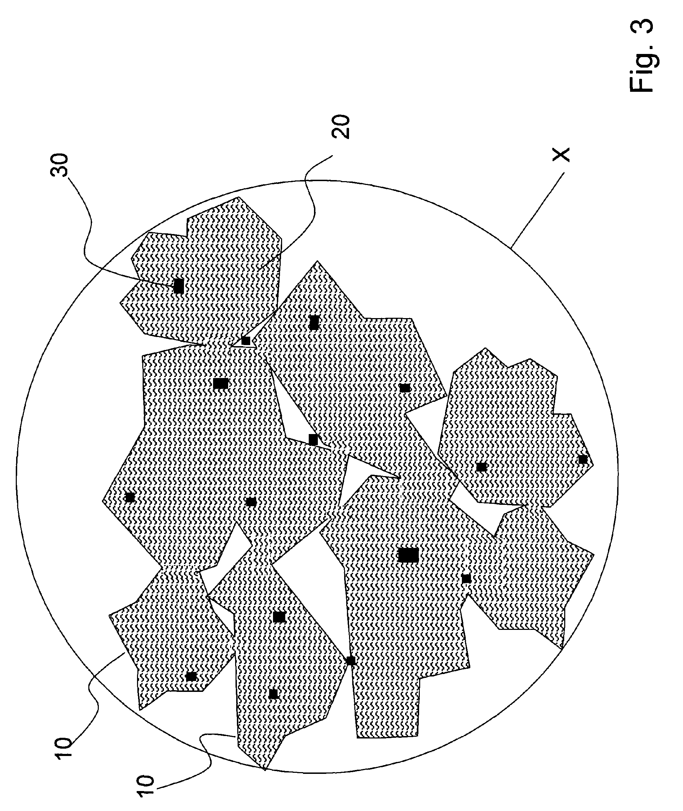

In Figur 3 ist ein vergrößerter Ausschnitt X aus dem entstehenden Gefüge des Formkörpers bei schematisch dargestellt. Körner 10 sind über Sinterbrücken miteinander zu einem stabilen Festkörper verbunden. Die Körner umfassen amorphes Quarzglas 20. In das Gefüge sind SiO₂-Kristalle 30 eingebettet. Diese können im Innern eines Korns vorliegen und/oder an Stellen, an denen Körner aneinanderstoßen und/oder in Randbereichen der Körner, die in Hohlräume des Gefüges übergehen.

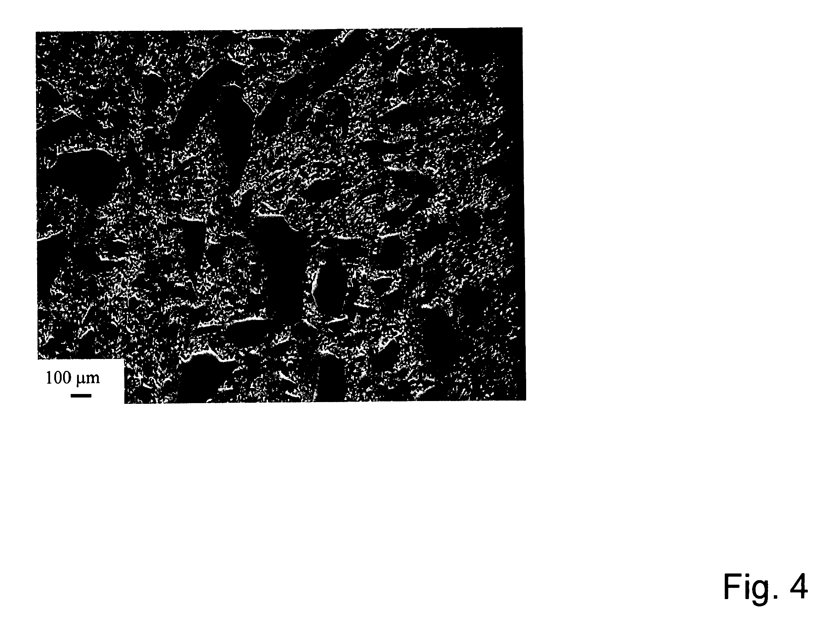

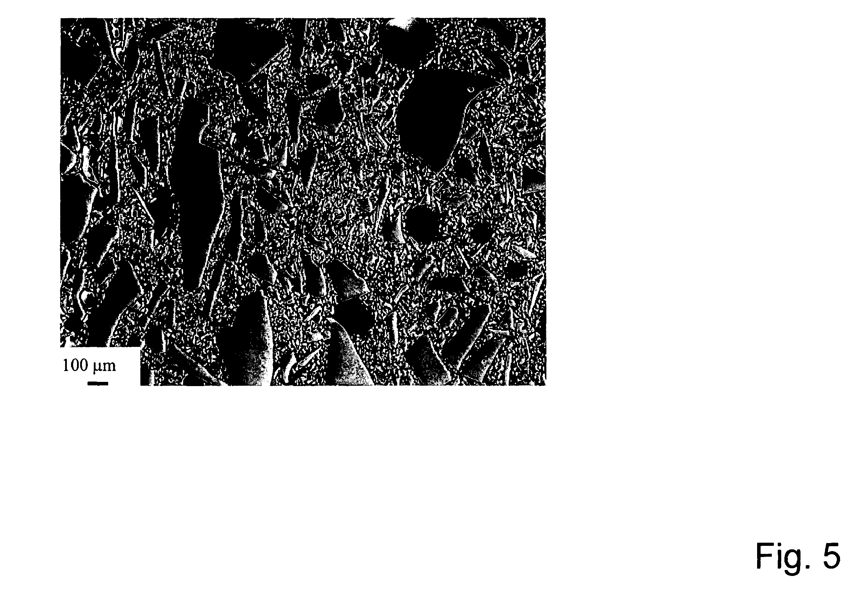

In Figur 4 ist die Mikrostruktur eines Gefüges nach der Sinterung im Temperaturbereich mit beginnender Christobalitbildung im Schliffbild dargestellt. Die Rohdichte des Materials beträgt 1,95 g/cm³; seine offene Porosität liegt bei 11,0 %. Der Christobalitanteil selbst lässt sich dabei im Schliffbild optisch nicht herausarbeiten. Zum Vergleich ist in Figur 5 die Mikrostruktur eines Gefüges im Schliffbild gezeigt, wobei das Gefüge nach Standardsinterung hergestellt wurde. Die Rohdichte dieses Materials beträgt 1,84 g/cm³; seine offene Porosität liegt bei 13,3 %.

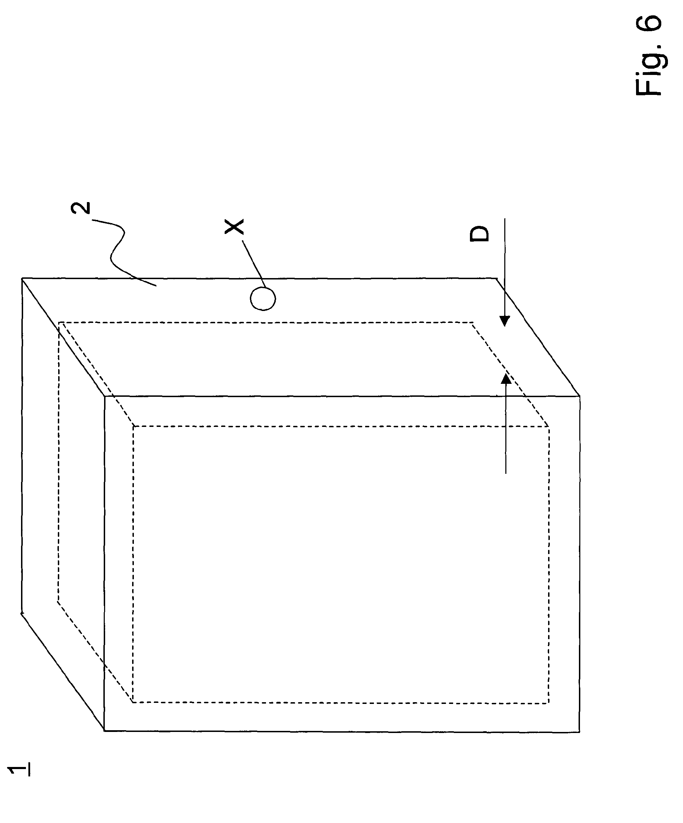

In Figur 6 ist ein Formkörper 1 schematisch dargestellt. Der Formkörper 1 hat einen Randbereich 2 mit einer Dicke D. Zumindest im Randbereich 2 weist der Formkörper 1 ein Gefüge auf, wie es in Figur 3 illustriert ist.

Der Formkörper weist eine bessere Korrosionsbeständigkeit gegenüber Glasschmelzen auf, weil durch die erste Cristobalit-Bildung schon bei der Herstellung die Umwandlung im späteren Einsatz schneller startet. Cristobalit ist beständiger als Korrosion durch Glas als Kieselglas.

Zur Bestimmung der Korrosionsbeständigkeit wurde folgender Korrosionstest gegenüber Borosilikatglas 3.3 gemäß ISO 3585 und EN 1595 durchgeführt. Es wurde Glas mit folgender Zusammensetzung verwendet:
81 % Kieselsäure (SiO₂),
13 % Borsäure (B₂O₃),
4 % Alkalien (Na₂O K2O) und
2 % andere Bestandteile (zum Beispiel Al₂O₃).

In einem statischen Korrosionstest werden Materialproben des erfindungsgemäßen Formkörpers in die Glasschmelze eingehängt. Dabei werden weder die Glasschmelze noch die Materialprobe bewegt. Die Versuchsdauer beträgt 168 Stunden bei einer Versuchstemperatur von 1400°C. Nach Ablauf der Versuchsdauer wird der Abtrag von der Oberfläche der Materialproben gegenüber den Abmessungen der Probe zu Beginn des Versuchs gemessen.

An einer Probe mit einem Anteil an kristallinem SiO₂ ist der Abtrag dabei geringer als bei einer Probe aus herkömmlichen Material, das zu Beginn des Versuchs keinen Anteil an kristallinem SiO₂ aufweist. Bei der Temperatur von 1400°C liegt das kristalline in Form von Cristobalit vor.

An einer cristobalithaltigen Probe beträgt der Abtrag im Mittel 0,77 mm, im einzelnen wurden Werte zwischen 0,71 mm und 0,82 mm gemessen. Der Abtrag an einer Vergleichsprobe ohne Cristobalitanteil beim Start des Versuchs beträgt im Mittel 1,05 mm, im einzelnen wurden Werte zwischen 1,0 mm und 1,1 mm gemessen.

Bei der Temperatur von 1400°C bildet sich auch in der Vergleichsprobe Cristobalit. Die Probe des erfindungsgemäßen Materials weist jedoch schon unmittelbar zu Beginn des Versuchs einen Cristobalitanteil an der Oberfläche auf. Am Ende des Versuchs ist das Material beider Proben nahezu vollständig in Cristobalit umgewandelt, der Restglasgehalt beträgt weniger als 10 %.

Außer der erhöhten Korrosionsbeständigkeit ist durch die Erfindung auch der Herstellungsprozeß vereinfacht, da eine höhere Sintertemperatur genutzt werden kann. Dies bringt eine einfachere Ofenregelung mit sich, zudem kann die Brenndauer aufgrund der höheren Temperaturen verkürzt werden. Da die Brenntemperatur bei der Herstellung der Bauteile erhöht und somit eine bessere Versinterung erzielt wird, werden die Restporosität vermindert und die Dichte sowie die mechanische Stabilität der Bauteile erhöht.

Zudem ist die Schwindung aufgrund von Nachsintereffekten im späteren Einsatz geringer. Liegen die Einsatztemperaturen über den Brenntemperaturen, so kann das Material aufgrund des weitergehenden Sinterprozesses schwinden. Da gemäß der Erfindung bereits durch den Herstellungsprozeß alleine eine höhere Rohdichte beziehungsweise geringere Restporosität des Materials erreicht wird, ist diese Schwindung gegenüber bekannten Materialien bei dem erfindungsgemäßen Material deutlich vermindert.

Die Schwindung im späteren Einsatz liegt für ein Material mit einer Sinterung ohne beginnende Christoballitbildung im Bereich von 2,0 % bis 4 %. Die Schwindung ist sehr stark beeinflusst von der Korngrößenverteilung des Ausgangsmaterials zur Herstellung des Schlickers und von der Brenntemperatur. Wird die Brenntemperatur -wie oben beschrieben- so hoch gewählt, dass der Bereich der Christoballit-Bildung erreicht wird, lässt sich die Schwindung auf 1,0 bis 2,0 % reduzieren.

### Beispiel für ein Material mit folgender Korngrößenverteilung des Ausgangsrohstoffes:

| Anteil [Gew-%] | Korngröße |
|---|---|
| | |
| 40 bis 60 | kleiner als 0,3 mm |
| 20 bis 30 | 0,3 mm bis 1,0 mm |
| 20 bis 30 | 1,0 mm bis 6,0 mm |

Bei einem normalen Sintern ohne Erreichen der Christobalit-Bildungszone liegt die Schwindung im Mittel zwischen 2,5 % und 3 %. Durch ein Sintern mit beginnender Christobalitbildung lässt sich die Schwindung auf weniger als 1,5 % (zum Beispiel auf 1,0 bis 1,5%) reduzieren.

Die dichtere Versinterung führt auch zu einer höheren mechanischen Festigkeit des Materials und zu einer besseren Wärmeleitfähigkeit. Für ein Material mit oben genannter Korngrößenverteilung im Ausgangsrohstoff ergeben sich folgende Werte:

| | Material ohne Christobalit | versintert mit beginnender Christobalitbildung |
|---|---|---|
| Kaltbiegefestigkeit (Raumtemperatur) [MPa] | 14 | 23 |
| | | |
| | Material ohne Christobalit | versintert mit beginnender |

| | | Christobalitbildung |
|---|---|---|
| Elastizitätsmodul (Raumtemperatur) [GPa] | 18 | 30 |
| Heißbiegefestigkeit (900°C) [MPa] | 20 | 46 |
| Elastizitätsmodul (bei 900°C) [GPa] | 26 | 47 |
| Wärmeleitfähigkeit [W/mK] bei 150°C | 1,04 | 1,4 |
| Wärmeleitfähigkeit [W/mK] bei 550°C | 1,24 | 1,6 |
| Dichte [kg/m³] | 1960 | 2015 |

Es handelt sich um Durchschnittswerte aus mehreren Untersuchungen. Einzelwerte können davon abweichen. Die Wanddicke des Körpers, aus dem die Proben herausgearbeitet wurden, betrug 40 mm.

Es ist dem Fachmann ersichtlich, dass die Erfindung nicht auf die vorstehend beschriebenen Ausführungsbeispiele beschränkt ist, sondern vielmehr in vielfältiger Weise variiert werden kann. Insbesondere können die Merkmale der einzelnen Ausführungsbeispiele auch miteinander kombiniert werden.

## Patentansprüche

1. Verfahren zum Herstellen eines Formkörpers mit den Schritten:
a) Bereitstellen von Quarzglas in Form von im wesentlichen amorphen SiO₂-Körnern, von denen maximal 5 %, bevorzugt maximal 3 %, besonders bevorzugt maximal 2 % einen Durchmesser größer als 15 mm, bevorzugt größer als 10 mm, besonders bevorzugt größer als 6 mm aufweisen,
b) Zugabe von Wasser zu den Quarzglaskörnern zum Herstellen einer Schlickermasse,
c) Gießen der Schlickermasse in eine Form, die einen Hohlkörper mit der inversen Gestalt des herzustellenden Formkörpers umfaßt,
d) Trocknen der Schlickermasse zu einem Vor-Körper,
e) Sintern des Vor-Körpers bei einer Sintertemperatur, bei welcher zumindest ein Teil des Quarzglases von der amorphen in eine kristalline Modifikation, insbesondere in beta-Cristobalit, umgewandelt wird,
f) Abkühlen des gesinterten Formkörpers auf eine Temperatur unter 300°C, bevorzugt unter 270°C, derart, daß ein Gefüge entsteht, welches kristallines SiO₂ enthält.

2. Verfahren nach Anspruch 1,
**dadurch gekennzeichnet, daß**
in Schritt a) im wesentlichen reines Quarzglas in amorpher Form mit einem Gehalt von mindestens 99,0 Gew.-%, bevorzugt mindestens 99,5 Gew.-%, besonders bevorzugt mindestens 99,9 Gew.-% an SiO₂ eingesetzt wird.

3. Verfahren nach einem der vorangegangenen Ansprüche,
**dadurch gekennzeichnet, daß**
in Schritt b) im wesentlichen alkalifreies Wasser verwendet wird.

4. Verfahren nach einem der vorangegangenen Ansprüche,
**dadurch gekennzeichnet, daß** das Sintern des Vor-Körpers in Schritt e) bei einer Temperatur von mindestens 1080°C, vorzugsweise von mindestens 1120°C, bevorzugt von mindestens 1140°C, besonders bevorzugt von mindestens 1180 °C durchgeführt wird.

5. Verfahren nach einem der vorangegangenen Ansprüche,
**dadurch gekennzeichnet, daß**
die maximale Temperatur beim Sintern in Schritt e) 1285°C beträgt, insbesondere bei einer größten Wandstärke des Vor-Körpers von kleiner oder gleich 85 mm.

6. Verfahren nach einem der vorangegangenen Ansprüche,
**dadurch gekennzeichnet, daß**
die maximale Temperatur beim Sintern in Schritt e) 1250°C beträgt, insbesondere bei einer größten Wandstärke des Vor-Körpers von mehr als 85 mm.

7. Formkörper, insbesondere herstellbar oder hergestellt mit einem Verfahren nach einem der Ansprüche 1 bis 6,
**dadurch gekennzeichnet, daß**
der Formkörper mindestens 99,0 mol.-%, bevorzugt mindestens 99,5 mol.-%, besonders bevorzugt mindestens 99,9 mol.-% an SiO₂ umfaßt.

8. Formkörper nach Anspruch 7,
**dadurch gekennzeichnet, daß**
der Formkörper in seiner äußeren Schicht, welche von seiner Oberfläche aus gemessen eine Dicke von bis zu 40 mm aufweist, einen über die Dicke gemittelten Gehalt im Bereich von 2 Vol-% bis 5 Vol-%, bevorzugt von 2,5 Vol-% bis 4,5 Vol-%, besonders bevorzugt von 2,9 Vol.-% ± 0,1 Vol.-% kristallines SiO₂, insbesondere Cristobalit umfaßt.

9. Formkörper nach einem der vorangegangenen Ansprüche,
**dadurch gekennzeichnet, daß**
der Formkörper in seiner äußeren Schicht, welche von seiner Oberfläche aus gemessen eine Dicke von bis zu 20 mm aufweist, einen über die Dicke gemittelten Gehalt im Bereich von 2,3 Vol-% bis 5,5 Vol-%, bevorzugt im Bereich von 3,0 Vol-% bis 5,5 Vol-%, besonders bevorzugt von 3,5 Vol-% bis 4,5 Vol-%, besonders bevorzugt von 3,75 Vol.-% ± 0,1 Vol.-% kristallines SiO₂, insbesondere Cristobalit, umfaßt.

10. Formkörper nach einem der vorangegangenen Ansprüche,
**dadurch gekennzeichnet, daß**
der Formkörper in seiner äußeren Schicht, welche von seiner Oberfläche aus gemessen eine Dicke von bis zu 10 mm aufweist, einen über die Dicke gemittelten Gehalt im Bereich von 2,3 Vol-% bis 6,5 Vol-%, bevorzugt im Bereich von 3,5 Vol-% bis 6,5 Vol-%, besonders bevorzugt von 4 Vol-% bis 5,3 Vol-%, besonders bevorzugt von 4,6 Vol.-% ± 0,1 Vol.-% kristallines SiO₂, insbesondere Cristobalit umfaßt.

11. Formkörper nach einem der vorangegangenen Ansprüche,
**dadurch gekennzeichnet, daß**
der Formkörper in seiner äußeren Schicht, welche von seiner Oberfläche aus gemessen eine Dicke von bis zu 5 mm aufweist, einen über die Dicke gemittelten Gehalt im Bereich von 2,3 Vol-% bis 7,5 Vol-%, bevorzugt im Bereich von 4,0 Vol-% bis 7,5 Vol-%, besonders bevorzugt von 4,5 Vol-% bis 6,5 Vol-%, besonders bevorzugt von 5,1 Vol.-% ± 0,1 Vol.-% kristallines SiO₂, insbesondere Cristobalit umfaßt.

12. Formkörper nach einem der vorangegangenen Ansprüche,
**dadurch gekennzeichnet, daß**
der Formkörper in seiner äußeren Schicht, welche von seiner Oberfläche aus gemessen eine Dicke von bis zu 2 mm aufweist, einen über die Dicke gemittelten Gehalt im Bereich von 2,3 Vol-% bis 8,0 Vol-%, bevorzugt im Bereich von 4,3 Vol-% bis 8,0 Vol-%, besonders bevorzugt von 4,8 Vol-% bis 6,8 Vol-%, besonders bevorzugt von 5,3 Vol.-% ± 0,1 Vol.-% kristallines SiO₂, insbesondere Cristobalit umfaßt.

13. Formkörper nach einem der vorangegangenen Ansprüche,
**dadurch gekennzeichnet, daß**
der Formkörper in seiner äußeren Schicht, welche von seiner Oberfläche aus gemessen eine Dicke von bis zu 1 mm aufweist, einen über die Dicke gemittelten Gehalt im Bereich von 2,5 Vol-% bis 8,5 Vol-%, bevorzugt im Bereich von 4,5 Vol-% bis 8 Vol-%, besonders bevorzugt von 5 Vol-% bis 6,3 Vol-%, besonders bevorzugt von 5,4 Vol.-% ± 0,1 Vol.-% kristallines SiO₂, insbesondere Cristobalit umfaßt.

14. Formkörper nach einem der vorangegangenen Ansprüche,
**dadurch gekennzeichnet, daß**
der Formkörper in seiner äußeren Schicht, welche von seiner Oberfläche aus gemessen eine Dicke von bis zu 0,3 mm aufweist, einen über die Dicke gemittelten Gehalt im Bereich von 2,7 Vol-% bis 9,5 Vol-%, bevorzugt im Bereich von 4,7 Vol-% bis 9,5 Vol-%, besonders bevorzugt von 5,1 Vol-% bis 6,8 Vol-%, besonders bevorzugt von 5,5 Vol.-% ± 0,1 Vol.-% kristallines SiO₂, insbesondere Cristobalit umfaßt.

15. Formkörper nach einem der vorangegangenen Ansprüche,
**dadurch gekennzeichnet, daß**
der Formkörper in einer Schicht, welche von seiner Oberfläche aus gemessen zwischen einer Tiefe von 20 mm und einer Tiefe von bis zu 40 mm liegt und somit eine Dicke von bis zu 20 mm aufweist, einen über die Dicke gemittelten Gehalt im Bereich von 1 Vol-% bis 3 Vol-%, bevorzugt von 1,5 Vol-% bis 2,5 Vol-%, besonders bevorzugt von 2 Vol.-% ± 0,1 Vol.-% kristallines SiO₂, insbesondere Cristobalit umfaßt.

16. Formkörper nach einem der vorangegangenen Ansprüche,
**dadurch gekennzeichnet, daß**
der Formkörper zumindest in einem Bereich, insbesondere in einem Bereich seiner äußeren Schicht, welche von der Oberfläche des Formkörpers aus gemessen eine Dicke von bis zu 20 mm aufweist, eine über die Dicke gemittelte Dichte im Bereich von 1,92 g/cm³ bis 2,12 g/cm³ aufweist.

17. Formkörper nach einem der vorangegangenen Ansprüche,
**dadurch gekennzeichnet, daß**
der Formkörper zumindest in einem Bereich, insbesondere in einem Bereich seiner äußeren Schicht, welche von der Oberfläche des Formkörpers aus gemessen eine Dicke von bis zu 20 mm aufweist, eine über die Dicke gemittelte offene Porosität im Bereich von weniger als 12 %, bevorzugt von weniger als 8 %, besonders bevorzugt von weniger als 6 %, besonders bevorzugt von 5,5 % aufweist.

18. Verwendung eines Formkörpers nach einem der Ansprüche 7 bis 17 zumindest als Teil eines Tiegels und/oder einer Schmelzwanne und/oder einer Läuterwanne und/oder einer Rohrleitung und/oder eines Rührers und/oder eines Aggregats zum Einsatz in Kontakt mit einer Schmelze zumindest eines Metalls und/oder Halbmetalls und/oder Halbleiters und/oder Glases.
